Europäisches Patentamt

European Patent Office

Office européen des brevets

(19)

(11) **EP 1 121 099 B1**

(12) # FASCICULE DE BREVET EUROPEEN

(45) Date de publication et mention
de la délivrance du brevet:
**21.04.2004 Bulletin 2004/17**

(21) Numéro de dépôt: **99947537.9**

(22) Date de dépôt: **12.10.1999**

(51) Int Cl.⁷: **A61K 9/00**, A61K 9/20

(86) Numéro de dépôt international:
**PCT/FR1999/002443**

(87) Numéro de publication internationale:
**WO 2000/023045 (27.04.2000 Gazette 2000/17)**

(54) **COMPOSITION PHARMACEUTIQUE A RESIDENCE GASTRIQUE ET A LIBERATION CONTROLEE**

IM MAGEN VERWEILENDES ARZNEIMITTEL MIT VERZÖGERTER WIRKSTOFFABGABE

PHARMACEUTICAL COMPOSITION WITH GASTRIC RESIDENCE AND CONTROLLED RELEASE

(84) Etats contractants désignés:
**AT BE CH CY DE DK ES FI FR GB GR IE IT LI LU
MC NL PT SE**
Etats d'extension désignés:
**AL LT LV MK RO SI**

(30) Priorité: **16.10.1998 FR 9812977**

(43) Date de publication de la demande:
**08.08.2001 Bulletin 2001/32**

(73) Titulaire: **SANOFI-SYNTHELABO
75013 Paris (FR)**

(72) Inventeurs:
• **ALAUX, Gérard
F-78650 Beynes (FR)**
• **ANDRE, Frédéric
F-92160 Antony (FR)**
• **CUINE, Alain
F-77310 Saint Fargeau-Ponthierry (FR)**
• **LEWIS, Gareth
F-91410 Dourdan (FR)**

(74) Mandataire: **Thouret-Lemaitre, Elisabeth
Sanofi-Synthélabo
Service Brevets
174, avenue de France
75013 Paris (FR)**

(56) Documents cités:
EP-A- 0 669 129          WO-A-96/29054
WO-A-97/47285          WO-A-98/11879
FR-A- 2 762 213

EP 1 121 099 B1

**Description**

La présente invention a pour objet des compositions pharmaceutiques à résidence gastrique et à libération contrôlée.

La demande de brevet EP 669 129 est relative à des compositions pharmaceutiques à temps de résidence gastrique prolongé comportant à titre de polymère gonflant un mélange de polymères contenant des groupes lactames et de polymères contenant des groupes carboxy.

La demande de brevet WO97/47285 concerne des compositions pharmaceutiques à résidence gastrique contenant une matrice solide consistant en un polymère gonflant et un agent chimique capable par action pharmacologique de retenir la substance active dans l'estomac.

La demande de brevet WO96/29054 décrit des compositions pharmaceutiques destinées à la prévention du reflux gastrique comprenant une pectine bien définie associée à un composant contenant des ions calcium et un agent formant du gaz.

On cherche le plus souvent à administrer les médicaments par voie orale. Cependant, l'administration par voie orale est parfois rendue difficile dans les cas où le principe actif a une faible biodisponibilité.

On entend ici par biodisponibilité la fraction de principe actif qui est absorbée depuis sa forme pharmaceutique et qui parvient dans le plasma.

D'autres principes actifs sont absorbés et peuvent donc être administrés par voie orale, mais leur absorption est incomplète et parfois irrégulière. Certains autres principes actifs sont bien absorbés à partir de formes pharmaceutiques à libération rapide, le principe actif étant alors libéré en moins d'une demi-heure, mais sont moins bien absorbés à partir de formes pharmaceutiques à libération prolongée.

Une telle biodisponibilité faible et irrégulière peut être le résultat de plusieurs facteurs. Parmi eux on peut citer une faible solubilité ou une dissolution très lente du principe actif, une dégradation chimique ou enzymatique du principe actif dans le tractus gastro-intestinal ou une absorption lente ou incomplète du principe actif.

En effet, un certain nombre de principes actifs, bien que suffisamment solubles, sont peu absorbés au niveau colonique ou moins absorbés à ce niveau qu'au niveau des parties hautes de l'intestin grêle, à savoir le duodénum, le jéjunum et l'iléum.

D'autre part, une forme à libération prolongée est utile pour beaucoup de médicaments, par exemple pour permettre une administration moins fréquente : 1 fois par jour au lieu de 2 fois ou 2 fois par jour au lieu de 3 fois.

Lorsque le principe actif est absorbé dans les régions basses du tractus gastro-intestinal de façon lente ou incomplète, la conception d'une forme à libération prolongée, qui typiquement devrait libérer le principe actif pendant 12 à 16 heures devient difficile. Le problème devient d'autant plus difficile s'il existe une fenêtre d'absorption, c'est à dire que le principe actif est bien absorbé seulement dans une partie du tractus gastro-intestinal. Par exemple le principe actif peut être bien absorbé seulement au niveau duodénal et jéjunal.

En effet, une forme pharmaceutique à libération prolongée nécessite un temps de libération d'au moins 8 heures, qui n'est pas atteint dans le cas d'un principe actif à absorption essentiellement au niveau des parties hautes de l'intestin grêle. C'est le problème que se propose de résoudre la demanderesse.

La présente invention vise donc à ralentir la vitesse de passage gastro-intestinal et donc à augmenter le temps disponible pour l'absorption au niveau des parties hautes de l'intestin grêle et plus spécifiquement duodénum, jéjunum et iléum, tout en contrôlant le profil de libération.

L'invention consiste ainsi en une composition pharmaceutique à résidence gastrique, caractérisée en ce qu'elle comporte deux ou trois couches et en ce qu'elle comprend :

(a) un principe actif associé à un excipient modifiant sa libération,

(b) un système générateur de dioxyde de carbone dans une matrice hydrophile polymérique gonflante.

Les comprimés à deux ou trois couches réalisés à partir des différentes combinaisons de (a) et de (b) font partie de l'invention, (a) et (b) pouvant être compris dans une même couche [(a)+(b)] ou dans des couches distinctes [(a)] et [(b)]. Les couches redondantes [(a)], [(b)] ou [(a)+(b)] dans un même comprimé peuvent posséder des compositions et des dimensions différentes.

Font également partie de l'invention les compositions à résidence gastrique à deux ou trois couches comprenant (a) et (b), caractérisées en ce qu'elles comportent une couche soluble et/ou érodable. Le comprimé peut ainsi comporter une couche [(a)+(b)] et une couche soluble et/ou érodable pour donner un comprimé bi-couche ou bien une couche soluble et/ou érodable recouverte de deux couches extérieures [(a)+(b)] pour donner un comprimé triple-couche.

Ce mode de réalisation permet, comme toutes les compositions selon l'invention, d'obtenir une augmentation graduelle de la surface de contact entre le comprimé et les liquides contenus dans l'estomac afin de tendre vers un profil de dissolution d'ordre zéro, à savoir un profil de libération contrôlé.

Les compositions selon l'invention sont caractérisées par le fait qu'au contact avec le suc gastrique, le(s) couche(s) [(b)] ou [(a)+(b)] augmentent de volume grâce au gonflement de la matrice polymérique hydrophile et la production immédiate en dioxyde de carbone. De cette façon la flottaison est obtenue rapidement et le temps de résidence gastrique obtenu est important.

Les compositions pharmaceutiques selon l'invention peuvent par exemple être utiles pour les benzamides et les $\alpha_1$-antagonistes, ainsi que les principes actifs suivants : le captopril, le furosémide, l'acide ursodésoxycholique, l'amoxicilline, le (+)-$\alpha$-aminométhyl-2-méthoxy-5-sulfonamidobenzèneméthanol (divulgué dans la demande de brevet EP 842 148 à l'exemple 3.6) ou le 3'-(2-amino-1-hydroxyéthyl)-4'-fluorométhanesulfonanilide (NS 49).

Les benzamides sont en particulier le métoclopramide, le véralipride, l'alizapride, le clébopride et plus particulièrement en l'amisulpride, le tiapride, le sulpiride et leurs sels.

Les $\alpha_1$-antagonistes sont en particulier la terazosine et l'alfuzosine sous forme d'énantiomère, diastéréoisomère ou de mélange en particulier de mélange racémique ou l'un de ses sels, en particulier le chlorhydrate d'alfuzosine. Ils sont destinés notamment au traitement de l'hypertrophie bénigne de la prostate.

Le captopril est utilisé notamment pour le traitement de l'hypertension, le furosémide comme diurétique, l'amoxicilline et ses sels comme antibiotique, et l'acide ursodésoxycholique et ses sels est utilisé pour le traitement des cholélithiases, désordres hépatiques et syphilis.

Au sens de la présente invention, les différents énantiomères ou diastéréoisomères des différents principes actifs ou familles de principes actifs (benzamides, $\alpha_1$-antagonistes) sont également couverts, y compris leurs mélanges, en particulier leurs mélanges racémiques, mais également leurs sels.

Parmi les principes actifs qui conviennent plus particulièrement aux compositions selon l'invention, on peut citer le (D)-tartrate d'amisulpride, le (S)-(-)-amisulpride, le (D)-tartrate du (S)-(-)-amisulpride, le chlorhydrate de tiapride, le chlorhydrate d'alfuzosine et le chlorhydrate de 3'-(2-amino-1-hydroxyéthyl)-4'-fluorométhanesulfonanilide.

La figure 1 représente trois modes de réalisation de l'invention avec différentes dispositions de (a) et (b).

La figure 2 représente le profil de dissolution du chlorhydrate de tiapride formulé dans un comprimé selon l'invention, à trois couches.

Le système générateur de dioxyde de carbone a pour principale fonction de former du dioxyde de carbone sous forme de bulles. Ces bulles contribuent à amener rapidement, puis à maintenir la composition pharmaceutique de l'invention à la surface des liquides contenus dans l'estomac.

Un système générateur de dioxyde de carbone convenant dans une composition pharmaceutique selon l'invention, comprend généralement au moins un agent générateur de dioxyde de carbone. L'agent générateur de dioxyde de carbone est habituellement un carbonate d'un métal alcalin ou alcalino-terreux, tel le carbonate de calcium ou un bicarbonate d'un métal alcalin, de préférence le bicarbonate de sodium.

Un tel système générateur de dioxyde de carbone, constitué seulement d'un agent générateur de dioxyde de carbone, ne commence à former des bulles de dioxyde de carbone qu'après avoir été mis en contact avec un milieu à pH acide, généralement celui de l'estomac.

Afin d'accélérer la formation des bulles de dioxyde de carbone, et donc d'améliorer la flottaison de la composition pharmaceutique à résidence gastrique de l'invention, on préfère mettre en oeuvre un système générateur de dioxyde de carbone indépendant du pH. Un tel système peut comprendre un agent générateur de dioxyde de carbone tel que ceux mentionnés plus haut, ainsi qu'au moins un composé acide choisi dans le groupe constitué par les acides monocarboxyliques comme l'acide lactique, les acides polycarboxyliques et les sels partiels d'acides polycarboxyliques. A titre de composés acides on peut plus particulièrement citer les acides tartrique, maléïque, malonique, malique, fumarique, succinique, adipique, citrique et leurs sels partiels, tel que le citrate monosodique.

Dans un tel système générateur de dioxyde de carbone, la teneur en composé acide est généralement choisie de sorte que le nombre de moles en ledit composé acide par rapport au nombre de moles en ledit agent générateur de dioxyde de carbone soit de 0,7 à 1,4 fois la stoechiométrie.

Toutefois, si le principe actif ou tout autre composant entrant dans la formulation de la composition selon l'invention présente un caractère basique, il peut être requis d'augmenter en conséquence la teneur en composé acide.

Les polymères hydrophiles convenant pour former une matrice hydrophile polymérique gonflante peuvent être choisis parmi :

- les polysaccharides naturels comme les alginates, la gomme de xanthane, la gomme de guar, la gomme arabique ou la gomme de caroube,
- les polysaccharides hémisynthétiques, en particulier les dérivés de la cellulose comme la méthylhydroxyéthylcellulose, la carboxyméthylcellulose et ses sels tels la carboxyméthylcellulose sodique ou la carboxyméthylcellulose calcique, l'hydroxypropylcellulose, hydroxypropylméthylcellulose ou
- les polymères hydrophiles synthétiques comme

    - les polyvinylpyrrolidones,

- les polymères dérivés des acides acrylique et méthylacrylique et leurs sels, tels les polyacrylates, notamment ceux commercialisés sous la marque Carbopol® ou
- les polymères d'acides aminés comme les polylysines.

Parmi les polysaccharides naturels on préfère les alginates et le gomme de xanthane.
Parmi les polysaccharides hémisynthétiques on préfère l'hydroxypropylcellulose et l'hydroxypropylméthylcellulose.

La matrice hydrophile polymérique gonflante est constituée d'un seul polymère hydrophile cité ci-dessus ou d'un mélange de plusieurs d'entre eux, choisis dans une même famille de polymères hydrophiles et préférentiellement jusqu'à trois d'entre eux.

Dans le cadre de la présente invention, les familles de polymères hydrophiles se définissent par la liste suivante :

- les polysaccharides naturels,
- les dérivés de la cellulose,
- les polyvynilpyrrolidones,
- les polymères dérivés des acides acrylique et méthacrylique et leurs sels,
- les polymères d'acides aminés.

Parmi les mélanges on peut notamment citer les mélanges d'hydroxypropylcellulose et d'hydroxypropylméthylcellulose et les mélanges d'hydroxypropylméthylcellulose de différents poids moléculaires.

Un mélange particulièrement préféré est consitué d'hydroxypropylméthylcellulose de différents poids moléculaires.

Afin de favoriser une augmentation rapide du volume de la composition pharmaceutique, avec les polymères hydrophiles précédemment cités, on utilise des produits et/ou excipients hydrophiles capables de favoriser l'hydratation des matrices polymériques gonflantes. On utilise à cet effet les diluants hydrophiles choisi parmi le lactose, le mannitol, le sorbitol ou la cellulose microcristalline, ou on introduit des substances qui permettent un mouillage plus rapide de la ou des matrices polymériques gonflantes, en facilitant de cette façon l'interaction entre les composants de cette ou ces couches et les fluides biologiques choisi parmi le laurylsulfate de sodium, le ricinoléate de sodium, le tetradecylsulfate de sodium, le dioctylsulfosulfonate de sodium, le cétomagrocol, le poloxamère ou les polysorbates,

On peut distinguer deux cas dans le choix des excipients modifiant la libération du principe actif compris dans (a) :

- Dans le cas où le principe actif et le système générateur de dioxyde de carbone sont dans la même couche [(a) +(b)], le ou les polymères hydrophiles qui forment la ou les matrices hydrophiles gonflantes jouent le rôle de modification de la libération du principe actif. Dès lors, un excipient spécifique modifiant la libération du principe actif n'est pas ajouté aux polymères hydrophiles gonflants.
- Dans le cas où le principe actif est dans une couche [(a)] ne comprenant pas (b), les excipients modifiant la libération du principe actif sont soit des polymères hydrophiles, soit des substances lipidiques qui peuvent former une matrice, soit une association des deux.

Les polymères hydrophiles pouvant modifier la libération du principe actif peuvent être choisis parmi ceux qui sont listés ci-dessus comme polymères hydrophiles formant une matrice gonflante, auxquels on peut ajouter l'éthylcellulose, la méthylcellulose, et les copolymères acryliques parmi lesquels ceux commercialisés sous la marque Eudragit®.

Les substances lipidiques peuvent être choisies parmi l'huile de ricin hydrogénée, la cire d'abeille, la cire de carnauba, le trimyristate de glycérol, le trilaurate de glycérol, le tristéarate de glycérol, le cetyl palmitate, et le béhénate de glycérol.

Le matériau soluble et/ou érodable dont une couche peut être constituée peut être choisi parmi : les diluants solubles tel que le lactose, le mannitol, le sorbitol, le xylitol, les polyalcools, parfois mélangés avec d'autres diluants hydrophiles tel que la cellulose microcristalline. Des polymères tels que l'hydroxyéthylcellulose, la carboxyméthylcellulose, l'alginate, l'albumine, l'amidon soluble et la gélatine peuvent être incorporés dans cette couche soluble et/ou érodable jusqu'à un pourcentage de 25% en poids pour contrôler la vitesse d'érosion et/ou solubilisation.

La réalisation technique des comprimés peut amener à introduire :

- des agents lubrifiants tels que le stéarate de magnésium, le stéarylfumarate de sodium, l'acide stéarique, le monostéarate de glycérol, les polyoxyethylèneglycols ayant un poids moléculaire de 400 à 7 000 000, l'huile de ricin hydrogénée, le béhénate de glycérol, les glycérides mono, bi- ou trisubstitués,
- des agents d'écoulement, tels que la silice colloïdale ou toute autre silice,
- et des liants, tampons, absorbants, ainsi que tout autre additif pharmaceutiquement acceptable.

Selon des modes de réalisation préférés, les compositions de l'invention peuvent prendre les différentes formes suivantes :

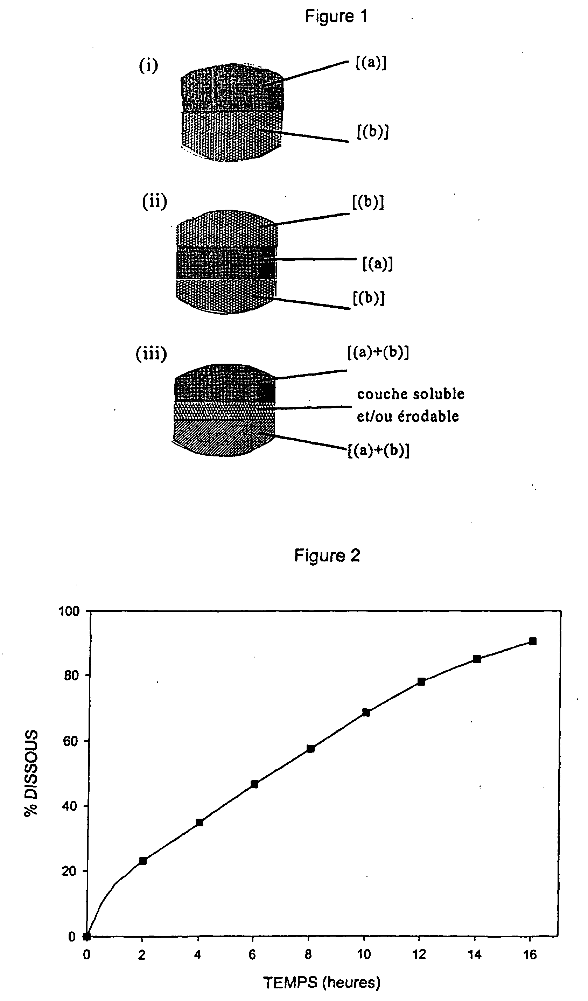

(1) un comprimé bi-couche, la première couche comprenant le principe actif et un excipient modifiant sa libération et la deuxième couche comprenant un générateur de dioxyde de carbone dans une matrice polymérique gonflante. Ce type de comprimé est représenté à la figure 1(i).

(2) un comprimé triple-couche, la première couche comprenant le principe actif et un excipient modifiant sa libération et les deux couches extérieures comprenant un générateur de dioxyde de carbone dans une matrice polymérique gonflante. La composition et la dimension des deux couches extérieures peuvent être identiques ou différentes.

Ce type de comprimé est représenté à la figure 1(ii).

(3) un comprimé triple-couche, les couches extérieures comprenant le principe actif associé à un excipient modifiant sa libération et un générateur de dioxyde de carbone, le tout dans une matrice polymérique gonflante et la couche intérieure étant constituée d'un matériau soluble et/ou érodable et éventuellement d'un générateur de dioxyde de carbone. La composition et la dimension des deux couches extérieures peuvent être identiques ou différentes.

Ce type de comprimé est représenté à la figure 1(iii).

Les comprimés de l'invention peuvent être produits de la manière suivante : on mélange des poudres et/ou des granulés en utilisant les technologies de production actuelles donc avec un procédé de production qui peut être immédiatement transféré sur le plan industriel.

Le comprimé pharmaceutique à deux ou trois couches est obtenu selon des procédés de compression très utilisés et connus par l'homme du métier.

Par exemple on peut produire les comprimés en utilisant des presses rotatives capables de produire des comprimés "multi-couches".

Normalement, la force de compression de travail varie de 7 à 50 kN (ou kilo newtons), et l'on obtient des comprimés à deux ou trois couches ayant une forme cylindrique, lenticulaire, sphéroïdale, ovoïdale, qui permettent une administration et une déglutition faciles.

Selon la quantité en principe actif qui est véhiculée chaque couche du comprimé peut avoir une épaisseur différente allant de 0,2 à 8 mm, mais de préférence de 1 mm à 4 mm.

A la composition pharmaceutique on peut en outre appliquer un enrobage en matériaux polymériques ayant pour but une simple protection de la composition pharmaceutique. L'enrobage doit alors être soluble en solution acide et neutre.

L'enrobage peut être appliqué par des méthodes classiques connues de l'homme du métier à l'aide de solutions organiques ou aqueuses.

Les teneurs en les différents composés constitutifs d'une composition pharmaceutique selon l'invention sont généralement choisies de sorte que la densité relative dans l'estomac de cette composition soit inférieure à 1,00.

Habituellement, une composition pharmaceutique selon l'invention comprend de 0,5 à 70 %, de préférence de 2 à 60 % en poids de principe actif, de 10 à 80 %, de préférence de 15 à 60 % en poids d'excipient modifiant la libération de principe actif, de 10 à 75 %, de préférence de 15 à 60 % en poids en au moins un polymère hydrophile et de 2,5 à 50 %, de préférence 10 à 40 % en poids d'agent générateur de dioxyde de carbone, les pourcentages étant exprimés par rapport au poids total de ladite composition.

Les exemples suivant illustrent la présente invention.

Exemple 1 : Comprimé flottant à libération prolongée à 3 couches de chlorhydrate de tiapride

Deux granulés sont préparés. Pour le granulé 1, Methocel® K100M, Avicel® PH102, et acide tartrique sont mélangés à sec, puis ils sont granulés avec de l'eau, dans un mélangeur granulateur, puis les granulés obtenus sont séchés. Les autres composants, stéarate de magnésium, Aerosil® 200, et carbonate monosodique sont ensuite ajoutés à sec, et mélangés. Pour le granulé 2, chlorhydrate de tiapride, Methocel®, et Avicel® sont mélangés à sec, puis ils sont granulés avec de l'eau, dans un mélangeur granulateur, puis les granulés obtenus sont séchés. Le stéarate de magnésium et l'Aerosil® sont ajoutés à sec, et mélangés. Des comprimés à 3 couches sont préparés, contenant 250 mg de granulé 1 dans la première couche extérieure, 280 mg de granulé 2 dans la couche intérieure, qui contient 100 mg tiapride base sous forme de chlorhydrate, et 200 mg granulé 1 dans la deuxième couche extérieure.

| Granulé 1 : couches extérieures 1 et 3 | |
|---|---|
| Methocel® K100M [1] | 45,6 % |
| Avicel® PH102 [2] | 15,3 % |
| Acide tartrique | 17,9 % |
| Carbonate monosodique | 20,0 % |
| Stéarate de magnésium | 1,0 % |
| Aérosil® 200 [3] | 0,2 % |
| | 100,0 % |
| **Granulé 2 : couche intérieure 2** | |
| Chlorhydrate de tiapride | 39,6 % |
| Methocel® K100M | 41,6 % |
| Avicel® PH101 | 17,6 % |
| Aérosil® 200 | 0,2 % |
| Stéarate de magnésium | 1,0 % |
| | 100,0 % |

[1] hydroxypropylméthylcellulose commercialisée par Dow Chemical Co.

[2] cellulose microcristalline commercialisée par Edward Mendell Co.

[3] silice colloïdale commercialisée par la société Degussa

La dissolution *in vitro* est testée selon la méthode suivante :

On utilise l'appareil à palettes décrit par la Pharmacopée européenne. L'agitation s'élève à 200 tpm. L'absorbance UV est lue en continu, grâce à un prélèvement par pompe péristaltique. Le pourcentage de tiapride dissous est déterminé en fonction du temps, par comparaison de l'absorbance UV à 288 nm de l'échantillon avec celui d'un étalon de tiapride chlorhydrate de concentration 0,222 mg/ml dans le milieu de dissolution. Le milieu de dissolution est constitué de 1000 ml d'acide chlorhydrique 0,01 M. Les résultats sont rapportés dans la figure 2.

On obtient une libération contrôlée du chlorhydrate de tiapride.

Exemple 2 : comprimé flottant à libération prolongée à 3 couches de NS49 sous forme de chlorhydrate

2 granulés sont préparés. Le granulé 1 est identique à celui de l'exemple précédent. Le granulé 2 est tel que décrit ci-dessous. Des comprimés à trois couches sont préparés, contenant 150 mg de granulé 1 dans la première couche extérieure, 100 mg de granulé 2 dans la couche intérieure qui contient 2 mg de NS 49 sous forme de chlorhydrate et 100 mg de granulé 1 dans la 2ème couche extérieure.

| Granulé 2 : couche intérieure 2 | |
|---|---|
| NS49 chlorhydrate | 2,0 % |
| Methocel® K100M | 45, 0 % |
| Avicel® PH101 | 51,8 % |
| Aérosil® 200 | 0,2 % |
| Stéarate de magnésium | 1,0 % |
| | 100,0 % |

**Revendications**

**1.** Composition pharmaceutique à résidence gastrique et à libération contrôlée, **caractérisée en ce qu'**elle comporte deux ou trois couches et **en ce qu'**elle comprend :

(a) un principe actif associé à un excipient modifiant sa libération,
(b) un système générateur de dioxyde de carbone dans une matrice hydrophile polymérique gonflante,

(a) et (b) pouvant être compris dans une même couche [(a)+(b)] ou dans des couches distinctes [(a)] et [(b)] et les couches redondantes [(a)], [(b)] ou [(a)+(b)] dans un même comprimé pouvant posséder des compositions et des dimensions différentes, **en ce que** la matrice polymérique gonflante est constituée d'un polymère hydrophile pouvant être choisi parmi les familles de polymère hydrophile suivantes :

- les polysaccharides naturels,
- les dérivés de la cellulose,
- les polyvinylpyrrolidones,
- les polymères dérivés des acides acrylique et méthacrylique et leurs sels,
- les polymères d'acides aminés

ou d'un mélange de 2 à 3 d'entre eux, choisis dans une même famille de polymères hydrophiles,
et **en ce que** la composition comprend en outre un excipient hydrophile capable de favoriser l'hydratation des matrices polymériques gonflantes choisi parmi le lactose, le mannitol, le sorbitol, la cellulose microcristalline, le laurylslfate de sodium, le ricinoléate de sodium, le tetradecylsulfate de sodium, le dioctylsulfosulfonate de sodium, le cétomagrocol, le poloxamère ou les polysorbates.

2. Composition selon la revendication 1, **caractérisée en ce que** les polymères hydrophiles peuvent être choisis parmi :

- les alginates, la gomme de xanthane, la gomme de guar, la gomme arabique ou la gomme de caroube,
- la méthylhydroxyéthylcellulose, la carboxyméthylcellulose, la caroboxyméthylcellulose sodique ou la carboxyméthylcellulose calcique, l'hydroxypropylcellulose, l'hydroxypropylméthylcellulose,
- les polyacrylates ou,
- les polylysines.

3. Composition selon la revendication 1 ou 2, **caractérisée en ce que** l'excipient modifiant la libération du principe actif peut être choisi parmi les polymères hydrophiles selon la revendication 2, le lactose, le mannitol, le sorbitol, la cellulose microcristalline, le laurylslfate de sodium, le ricinoléate de sodium, le tetradecylsulfate de sodium, le dioctylsulfosulfonate de sodium, le cétomagrocol, le poloxamère ou les polysorbates, ou parmi l'éthylcellulose, la méthylcellulose ou les copolymères acryliques, mais aussi, dans le cas où (a) et (b), tels que définis à la revendication 1, sont dans des couches distinctes, également parmi les substances lipidiques comme l'huile de ricin hydrogénée, la cire d'abeille, la cire de carnauba, le trimyristate de glycérol, le trilaurate de glycérol, le tristéarate de glycérol, le cetyl palmitate et le béhénate de glycérol ou une association d'un polymère hydrophile et d'une substance lipidique.

4. Composition selon l'une quelconque des revendications 1 à 3, **caractérisée en ce que** le système générateur de dioxyde de carbone comprend au moins un agent générateur de dioxyde de carbone pouvant être choisi parmi un carbonate d'un métal alcalin ou alcalino-terreux, tel le carbonate de calcium ou un bicarbonate d'un métal alcalin, tel le bicarbonate de sodium.

5. Composition selon la revendication 4, **caractérisée en ce que** le système générateur de dioxyde de carbone comprend au moins un agent générateur de dioxyde de carbone et au moins un composé acide choisi dans le groupe constitué par les acides monocarboxyliques, les acides polycarboxyliques et les sels partiels d'acides polycarboxyliques.

6. Composition selon l'une des revendications 4 ou 5, **caractérisée en ce que** le composé acide est l'acide tartrique, l'acide succinique, l'acide citrique ou l'un de leurs sels partiels tel que le citrate monosodique.

7. Composition selon l'une quelconque des revendications 1 à 6, **caractérisée en ce que** le principe actif est un benzamide, tel que métoclopramide, véralipride, alizapride, clébopride, amisulpride, tiapride ou sulpiride, sous forme d'énantiomère, de diastéréoisomères ou de mélange, en particulier de mélange racémique, ou l'un de ses sels.

8. Composition selon la revendication 7, **caractérisée en ce que** le benzamide est le (D)-tartrate d'amisulpride, le (S)-(-)-amisulpride, le (D)-tartrate du (S)-(-)-amisulpride ou le chlorhydrate de tiapride.

9. Composition selon l'une des revendications 1 à 6, **caractérisée en ce que** le principe actif est un $\alpha_1$-antagoniste tel que terazosine ou alfuzosine sous forme d'énantiomère, diastéréoisomère ou de mélange, en particulier de mélange racémique ou l'un de ses sels, en particulier le chlorhydrate d'alfuzosine.

**10.** Composition selon l'une des revendications 1 à 6, **caractérisée en ce que** le principe actif est le captopril, le furosémide, l'acide ursodésoxycholique ou l'amoxicilline, le (+)-α-aminométhyl-2-méthoxy-5-sulfonamidobenzèneméthanol ou le 3'-(2-amino-1-hydroxyéthyl)-4'-fluorométhanesulfonanilide ou l'un de leurs sels.

**11.** Composition selon la revendication 10, **caractérisée en ce que** le principe actif est le chlorhydrate de 3'-(2-amino-1-hydroxyéthyl)-4'-fluorométhanesulfonanilide.

**Patentansprüche**

**1.** Im Magen verweilende pharmazeutische Zubereitung mit gesteuerter Wirkstoff-Freisetzung, **dadurch gekennzeichnet, daß** sie zwei oder drei Schichten aufweist und daß sie folgendes umfaßt:

(a) einen Wirkstoff, der mit einem Trägermaterial verbunden ist, welcher seine Freisetzung modifiziert,

(b) ein System zur Kohlendioxidentwicklung in einer expandierbaren polymeren hydrophilen Matrix,

wobei (a) und (b) in ein und derselben Schicht [(a) + (b)] oder in getrennten Schichten [(a)] und [(b)] vorliegen können und die redundanten Schichten [(a)], [(b)] oder [(a) + (b)] in ein und derselben Tablette unterschiedliche Zusammensetzungen und Dimensionen aufweisen können, und daß die expandierbare polymere Matrix aus einem hydrophilen Polymer gebildet ist, welches aus den folgenden Familien von hydrophilen Polymeren ausgewählt sein kann:

- natürliche Polysaccharide,
- Cellulosederivate,
- Polyvinylpyrrolidone,
- von Acrylsäure und Methacrylsäure und deren Salzen abgeleitete Polymere,
- Polymere von Aminosäuren

oder einer Mischung aus 2 bis 3 davon, die aus einer gleichen Familien von hydrophilen Polymeren ausgewählt sind,
und daß die Zubereitung zusätzlich ein hydrophiles Trägermaterial enthält, welches dazu in der Lage ist, die Hydratation der expandierbaren polymeren Matrizes zu begünstigen, ausgewählt aus Lactose, Mannitol, Sorbitol, mikrokristalliner Cellulose, Natriumlaurylsulfat, Natriumricinoleat, Natriumtetradecylsulfat, Natriumdioctylsulfosulfonat, Cetromagrocol, Poloxamer oder Polysorbaten.

**2.** Zubereitung nach Anspruch 1, **dadurch gekennzeichnet, daß** die hydrophilen Polymere ausgewählt sein können aus:

- Alginaten, Xanthangummi, Guargummi, Gummi arabicum oder Caroubagummi,
- Methylhydroxyethylcellulose, Carboxymethylcellulose, Natriumcarboxymethylcellulose oder Calciumcarboxymethylcellulose, Hydroxypropylcellulose, Hydroxypropylmethylcellulose,
- Polyacrylaten oder
- Polylysinen.

**3.** Zubereitung nach Anspruch 1 oder 2, **dadurch gekennzeichnet, daß** das die Freisetzung des Wirkstoffs modifizierende Trägermaterial ausgewählt sein kann aus hydrophilen Polymeren gemäß Anspruch 2, Lactose, Mannitol, Sorbitol, mikrokristalliner Cellulose, Natriumlaurylsulfat, Natriumricinoleat, Natriumtetradecylsulfat, Natriumdioctylsulfosulfonat, Cetomagrocol, Poloxamer oder Polysorbaten, oder aus Ethylcellulose, Methylcellulose oder Acryl-Copolymeren,
jedoch auch, in dem Fall, da (a) und (b), wie sie in Anspruch 1 definiert sind, in getrennten Schichten vorliegen, aus Lipidsubstanzen, wie hydriertem Rizinusöl, Bienenwachs, Carnaubawachs, Glyceroltrimyristat, Glyceroltrilaurat, Glyceroltristearat, Cetylpalmitat, Glycerolbehenat oder einer Kombination aus einem hydrophilen Polymer und einer Lipidsubstanz.

**4.** Zubereitung nach einem der Ansprüche 1 bis 3, **dadurch gekennzeichnet, daß** das Kohlendioxid bildende System mindestens ein Kohlendioxid lieferndes Mittel umfaßt, welches aus einem Alkalimetall- oder Erdalkalimetallcarbonat, Calciumcarbonat oder einem Alkalimetallbicarbonat, wie Natriumbicarbonat, ausgewählt sein kann.

**5.** Zubereitung nach Anspruch 4, **dadurch gekennzeichnet, daß** das System zur Kohlendioxidentwicklung mindestens ein Kohlendioxid bildendes Mittel und mindestens eine saure Verbindung ausgewählt aus der aus Monocarbonsäuren, Polycarbonsäuren und Teilsalzen von Polycarbonsäuren gebildeten Gruppe umfaßt.

**6.** Zubereitung nach einem der Ansprüche 4 oder 5, **dadurch gekennzeichnet, daß** die saure Verbindung Weinsäure, Bernsteinsäure, Citronensäure oder eines ihrer Teilsalze, wie Mononatriumcitrat, ist.

**7.** Zubereitung nach einem der Ansprüche 1 bis 6, **dadurch gekennzeichnet, daß** der Wirkstoff ein Benzamid, wie Metoclopramid, Veraliprid, Alizaprid, Cleboprid, Amisulprid, Tiaprid oder Sulpirid, in Form des Enantiomeren, der Diastereoisomeren oder einer Mischung davon, insbesondere in Form einer racemischen Mischung, oder eines der Salze davon ist.

**8.** Zubereitung nach Anspruch 7, **dadurch gekennzeichnet, daß** das Benzamid Amisulprid-(D)-tartrat, (S)-(-)-Amisulprid, (S)-(-)-Amisulprid-(D)-tartrat oder Tiaprid-Hydrochlorid ist.

**9.** Zubereitung nach einem der Ansprüche 1 bis 6, **dadurch gekennzeichnet, daß** der Wirkstoff ein $\alpha_1$-Antagonist ist, wie Tetrazosin oder Alfuzosin in Form von Enantiomeren, Diastereoisomeren oder Mischungen davon, insbesondere der racemischen Mischung, oder eines der Salze davon, insbesondere Alfuzosin-Hydrochlorid.

**10.** Zubereitung nach einem der Ansprüche 1 bis 6, **dadurch gekennzeichnet, daß** der Wirkstoff Captopril, Furosemid, Ursodesoxycholin oder Amoxicillin, (+)-$\alpha$-Aminomethyl-2-methoxy-5-sulfonamidobenzolmethanol oder 3'-(2-Amino-1-hydroxyethyl)-4'-fluormethansulfonanilid oder eines der Salze davon ist.

**11.** Zubereitung nach Anspruch 10, **dadurch gekennzeichnet, daß** der Wirkstoff 3'-(2-Amino-1-hydroxyethyl)-4'-fluormethansulfonanilid-Hydrochlorid ist.

**Claims**

**1.** Controlled-release pharmaceutical composition with gastric residence, **characterized in that** it comprises two or three layers and **in that** it comprises:

    (a) an active principle combined with an excipient which modifies its release,
    (b) a carbon dioxide-generating system in a swelling hydrophilic polymer matrix,

(a) and (b) possibly being included in the same layer [(a)+(b)] or in separate layers [(a)] and [(b)] and the redundant layers [(a)], [(b)] or [(a)+(b)] in the same tablet possibly having different compositions and dimensions, **in that** the swelling polymer matrix consists of a hydrophilic polymer which may be chosen from the following families of hydrophilic polymers:

- natural polysaccharides,
- cellulose derivatives,
- polyvinylpyrrolidones,
- polymers derived from acrylic acid and methacrylic acid and salts thereof,
- aminoacid polymers,

or from a mixture of 2 or 3 of them, chosen from the same family of hydrophilic polymers,
and **in that** it also comprises a hydrophilic excipient capable of promoting the hydration of swelling polymer matrices, chosen from lactose, mannitol, sorbitol, microcrystalline cellulose, sodium lauryl sulphate, sodium ricinoleate, sodium tetradecyl sulphate, sodium dioctyl sulphosulphonate, ketomagrocol, poloxamer and polysorbates.

**2.** Composition according to Claim 1, **characterized in that** the hydrophilic polymers may be chosen from:

- alginates, xanthan gum, guar gum, gum arabic or carob gum,
- methylhydroxyethylcellulose, carboxymethylcellulose, sodium carboxymethylcellulose or calcium carboxymethylcellulose, hydroxypropylcellulose or hydroxypropylmethylcellulose,
- polyacrylates, or

- polylysines.

3. Composition according to Claim 1 or 2, **characterized in that** the excipient which modifies the release of the active principle may be chosen from the hydrophilic polymers according to Claim 2, lactose, mannitol, sorbitol, microcrystalline cellulose, sodium lauryl sulphate, sodium ricinoleate, sodium tetradecyl sulphate, sodium dioctyl sulphosulphonate, ketomagrocol, poloxamer and polysorbates, or from ethylcellulose, methylcellulose and acrylic copolymers, and also, when (a) and (b), as defined in Claim 1, are in separate layers, also from lipid substances such as hydrogenated castor oil, beeswax, carnauba wax, glyceryl trimyristate, glyceryl trilaurate, glyceryl tristearate, cetyl palmitate and glyceryl behenate, or a combination of a hydrophilic polymer and a lipid substance.

4. Composition according to any one of Claims 1 to 3, **characterized in that** the carbon dioxide-generating system comprises at least one carbon dioxide-generating agent which may be chosen from an alkali metal carbonate or alkaline-earth metal carbonate, such as calcium carbonate, and an alkali metal bicarbonate, such as sodium bicarbonate.

5. Composition according to Claim 4, **characterized in that** the carbon dioxide-generating system comprises at least one carbon dioxide-generating agent and at least one acidic compound chosen from the group consisting of monocarboxylic acids, polycarboxylic acids and partial salts of polycarboxylic acids.

6. Composition according to either of Claims 4 and 5, **characterized in that** the acidic compound is tartaric acid, succinic acid, citric acid or a partial salt thereof, such as monosodium citrate.

7. Composition according to any one of Claims 1 to 6, **characterized in that** the active principle is a benzamide, such as metoclopramide, veralipride, alizapride, clebopride, amisulpride, tiapride or sulpiride, in the form of an enantiomer, diastereoisomers or a mixture, in particular a racemic mixture, or a salt thereof.

8. Composition according to Claim 7, **characterized in that** the benzamide is amisulpride (D)-tartrate, (S)-(-)-amisulpride, (S)-(-)-amisulpride (D)-tartrate or tiapride hydrochloride.

9. Composition according to one of Claims 1 to 6, **characterized in that** the active principle is an $\alpha_1$-antagonist such as terazosine or alfuzosine in the form of an enantiomer, a diastereoisomer or a mixture, in particular a racemic mixture, or a salt thereof, in particular alfuzosine hydrochloride.

10. Composition according to one of Claims 1 to 6, **characterized in that** the active principle is captopril, furosemide, ursodeoxycholic acid or amoxicillin, (+)-$\alpha$-aminomethyl-2-methoxy-5-sulphonamidobenzenemethanol or 3'-(2-amino-1-hydroxyethyl)-4'-fluoromethanesulphonanilide, or a salt thereof.

11. Composition according to Claim 10, **characterized in that** the active principle is 3'-(2-amino-1-hydroxyethyl)-4'-fluoromethanesulphonanilide hydrochloride.

## Figure 1

(i)

[(a)]

[(b)]

(ii)

[(b)]

[(a)]

[(b)]

(iii)

[(a)+(b)]

couche soluble
et/ou érodable

[(a)+(b)]

## Figure 2